# EUROPÄISCHE PATENTSCHRIFT

(11) **EP 0 916 106 B1**
(45) Veröffentlichungstag und Bekanntmachung des Hinweises auf die Patenterteilung: **23.01.2002**
(21) Anmeldenummer: 97937414.7
(22) Anmeldetag: 29.07.1997
(51) Int. Cl.: G02B 23/16

(54) **ENDOSKOP MIT TROCKENMITTEL**
ENDOSCOPE WITH DRYING AGENT
ENDOSCOPE A AGENT DE SECHAGE

(30) Priorität: 29.07.1996 DE 19630634; 19.11.1996 DE 19647851
(43) Veröffentlichungstag der Anmeldung: 19.05.1999
(73) Patentinhaber: Karl Storz GmbH & Co. KG, 78532 Tuttlingen (DE)
(72) Erfinder: RUDISCHHAUSER, Jürgen, D-78532 Tuttlingen (DE); HÖFIG, Siegfried, D-78532 Tuttlingen (DE)
(74) Vertreter: Münich, Wilhelm, Dr.
(86) Internationale Anmeldenummer: DE9701603
(87) Internationale Veröffentlichungsnummer: WO9804947

(56) Entgegenhaltungen:
- DE-A- 3 708 124
- DE-A- 4 135 988
- DE-A- 19 507 205
- US-A- 3 740 114
- US-A- 4 538 593

## Beschreibung

### Technisches Gebiet

Die Erfindung bezieht sich auf ein Endoskop mit einer Endoskopoptik und einem Trockenmittel, das in den Einbauraum der Endoskopoptik eingebracht und in diesem fixiert ist.

### Stand der Technik

Endoskope und insbesondere in der Medizintechnik eingesetzte Endoskope sind prinzipiell fluiddichte Systeme. Aus einer Reihe von Gründen kann jedoch im Einbauraum der Endoskopoptik Feuchtigkeit auftreten:

Zum einen ist es möglich, daß aufgrund der Luftfeuchtigkeit in dem Raum, in dem der Zusammenbau des Endoskops erfolgt, eine Restfeuchte im Einbauraum des Endoskops verbleibt. Zum anderen kann durch geringfügige Undichtigkeiten in Verbindungsstellen, die an und für sich noch keine teure Reparatur des Endoskops erfordern, Feuchtigkeit in den Einbauraum eindringen.

Deshalb ist es bereits seit längerem bekannt, im Einbauraum der Endoskopoptik ein Trockenmittel vorzusehen, das diese Restfeuchte adsorbiert.

Für die Inkorporation des Trockenmittels in den Einbauraum der Endoskopoptik sind die verschiedensten Lösungen bekannt geworden:

So ist es bekannt, das Trockenmittel in loser Form im Einbauraum anzuordnen. Dies hat jedoch den Nachteil, daß beim Bewegen der Optik Geräusche auftreten und zudem durch Abrieb des Trockenmittels durch Bewegung Staubbildung auftreten kann. Der Staub kann die optischen Elemente belegen.

Aus der DE 37 08 124 C2 ist ein Endoskop mit einem hydroskopischen Element bekannt, bei dem das hydroskopische Element als flexibles Streifenmaterial ausgebildet und in einem speziell hierfür vorgesehenen Teil der Endoskopoptik zwischen dem Linsenträger und der Außenwand angebracht ist. Dieses bekannte Endoskop, von dem bei der Formulierung des Oberbegriffs des Patentanspruchs 1 ausgegangen worden ist, hat den Nachteil, daß ein Austausch des hydroskopischen Elements nur bei einer vollständigen Zerlegung des Endoskops möglich ist.

Deshalb ist in der DE 195 07 205 A1 vorgeschlagen worden, eine hydroskopische Substanz unter einem abnehmbaren Wandstück des Gehäuses anzuordnen, das mit einer Kupplung gasdicht mit der übrigen Gehäusewand verbunden ist. Diese Ausführungsform hat den Nachteil, daß das Endoskop eine weitere Öffnung aufweist, deren Fluiddichtigkeit gewährleistet sein muß.

### Darstellung der Erfindung

Der Erfindung liegt die Aufgabe zugrunde, ein Endoskop mit einer Endoskopoptik und einem Trockenmittel, das in den Einbauraum der Endoskopoptik eingebracht und diesem fixiert ist, derart weiterzubilden, daß das Trockenmittel sicher in dem Einbauraum fixiert ist, und eine hohe Effizienz der Adsorption von Feuchtigkeit aufweist, so daß ein Austauschen des Trockenmittels in der Regel nicht erforderlich ist.

Eine erfindungsgemäße Lösung dieser Aufgabe ist im Patentanspruch 1 angegeben. Weiterbildungen der Erfindung sind Gegenstand der Ansprüche 2ff..

Erfindungsgemäß liegt das Trockenmittel in Form einer Mehrzahl von Kugeln und/oder von stabförmigen Elementen vort, die in wenigstens eine Ausnehmung in dem Einbauraum eingesetzt sind, und mittels wenigstens eines Fixierelements fixiert sind, das die Kugeln bzw. die stabförmigen Elemente lediglich auf einem Teil ihrer Fläche berührt.

Die Erfindung geht dabei von der Erkenntnis aus, daß Endoskope aus Servicegründen zerlegbar sein müssen, so daß es nicht erforderlich ist, eine zusätzliche Öffnung für den Austausch des Trockenmittels vorzusehen.

Durch die fixierte Anbringung des Trockenmittels im Einbauraum der Optik ist es für einen Wechsel des Trockenmittels nicht erforderlich, das Endoskop über das bei einem normalen Service übliche Maß hinaus zu zerlegen. Durch die Anordnung des Trockenmittels im Bereich des Okulars (Anspruch 5) steht ausreichend Platz zur Verfügung, um so viel Trockenmittel unterzubringen, daß die Menge an Trokkenmittel für eine durchschnittliche Lebensdauer eines Endoskops ausreichend ist. Selbstverständlich kann das Trockenmittel bei Bedarf dennoch ausgetauscht werden.

Durch die Verwendung einer Mehrzahl von Kugeln und/oder stabförmigen Elementen mit bevorzugt zylindrischer Form erhält man eine große Oberfläche des Trockenmittels, so daß die Adsorption des Trockenmittels effektiv erfolgt.

Das Fixierelement hat die Aufgabe, das in Form von Kugeln oder Stäben mit bevorzugt zylindrischer Form vorliegende Trockenmittel einerseits sicher zu fixieren, andererseits aber einen ungehinderten Luftaustausch zu gewährleisten, so daß die in dem Einbauraum der Optik vorhandene Feuchte von dem Trockenmittel adsorbiert werden kann.

In den Ansprüchen 2 und 3 sind mögliche Ausgestaltungen des Fixierelements gekennzeichnet. Die im Anspruch 2 angegebene Ausgestaltung hat den Vorteil, daß die Verwendung eines O-Rings als Fixierelement den größten Teil der Oberfläche des Trockenmittels frei läßt, so daß die Feuchte problemlos adsorbiert werden kann. Darüberhinaus ist der Ersatz von O-Ringen bei einem Wechsel des Trockenmittels problemlos möglich.

Durch die Verwendung eines Behältnisses, dessen Wandung feuchtepermeabel ist (Anspruch 3) und die insbesondere aus einer Membran besteht, ist eine sichere Fixierung auch bei Erschütterungen etc. gewährleistet.

Im Anspruch 6 sind verschiedene Möglichkeiten für das Trockenmittel angegeben. Derartige Trockenmittel, die insbesondere als Molekularsieb wirken können, sind handelsüblich erhältlich.

Bei einer vorteilhaften Weiterbildung kann ferner in dem Einbauraum der Optik eine Einrichtung zur Messung der Feuchte vorgesehen sein (Anspruch 7). Anhand des Meßergebnisses kann die Bedienungsperson feststellen, ob die Feuchte im Einbauraum der Optik innerhalb zulässiger Grenzen liegt. Wird eine zu hohe Feuchte angezeigt, kann dies die Ursache haben, daß das Trockenmittel erschöpft ist und ausgetauscht werden muß, oder ob daß das Endoskop eine Undichtigkeit aufweist, durch die Feuchtigkeit in einem Maße eintritt, daß sie von dem Trockenmittel nicht mehr adsorbiert werden kann.

Dabei kann gemäß Anspruch 8 die Einrichtung zur Messung der Feuchte bevorzugt einen Farbindikator aufweisen, dessen Farbe bei einem bestimmten Feuchtigkeitsgrad reversibel oder irreversibel umschlägt, und der in einem Fenster von außen sichtbar ist. Derartige Farbindikatoren sind im Handel erhältlich.

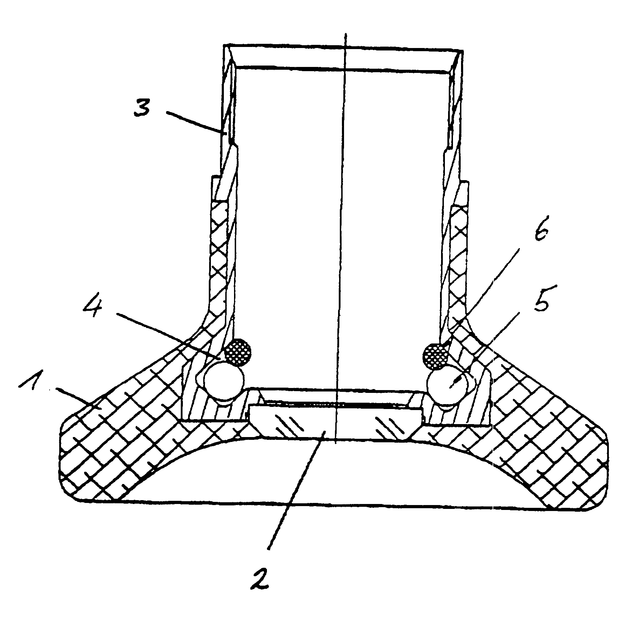

### Kurze Beschreibung der Zeichnung

Die Erfindung wird nachstehend ohne Beschränkung des allgemeinen Erfindungsgedankens anhand eines Ausführungsbeispielen unter Bezugnahme auf die einzige Figur der Zeichnung exemplarisch beschrieben, die zeigt:
- eine Querschnitt durch den proximalen Teil eines erfindungsgemäßen Endoskops.

### Darstellung eines Ausführungsbeispiels

Der in der Figur dargestellte proximale Teil eines erfindungsgemäßen Endoskops weist einen Okulartrichter 1 mit einem Okular-Deckglas 2 auf. Der Okulartrichter 1 ist in an sich bekannter Weise mit einem nur teilweise dargestellten Grundteil 3 des Endoskops verbunden. An dem Grundteil 3 befindet sich beispielsweise der nicht dargestellte Anschluß für ein Lichtleitkabel.

Im Bereich des Okular-Deckglases 2 ist im Grundteil 3 erfindungsgemäß eine umlaufende Nut 4 vorgesehen, in die Trockenmittel-Kugeln 5 eingelegt sind. Um die Kugeln 5 am Herausfallen aus der Nut 4 bei Bewegungen des Endoskops zu hindern, sind sie durch einen O-Ring 6 gehalten.

Diese Anordnung hat eine Reihe von Vorteilen:
- Die Trockenmittel-Kugeln 5 haben eine vergleichsweise große Oberfläche, die für die Adsorption zur Verfügung steht.
- Die Oberfläche ist "frei zugänglich", da der O-Ring die Kugeln nur auf einer vergleichsweise kleinen Fläche berührt.
- Auch bei vorhandenen Endoskopen ist ein Umbau auf die erfindungsgemäße Ausführungsform - durch Austausch des vergleichsweise billigen proximalen Teils oder durch nachträgliches Anbringen der Nut 4 - leicht möglich.
- Für die erfindungsgemäße Anordnung des Trockenmittels ist keine Vergrößerung des Endoskops erforderlich. Dennoch erfolgt keine Abschattung des Beobachtungsstrahlengangs.

Die bei einer bevorzugten Ausführungsform der Erfindung vorgesehene - in der Figur nicht dargestellte - Einrichtung zur Messung der Feuchte kann ebenfalls im Grundteil 3 vorgesehen sein. Das Fenster zur Beobachtung des Meßergebnisses und insbesondere zur Beobachtung des Farbindikators, dessen Farbe bei einem bestimmten Feuchtigkeitsgrad reversibel oder irreversibel umschlägt, muß natürlich so angeordnet sein, daß kein Licht in den Beobachtungsstrahlengang eintritt, das als störendes Streulicht wirken würde.

## Patentansprüche

1. Endoskop mit einer Endoskopoptik und einem Trockenmittel, das in den Einbauraum der Endoskopoptik eingebracht und in diesem fixiert ist,
**dadurch gekennzeichnet, daß** das Trockenmittel in Form einer Mehrzahl von Kugeln (5) und/oder von stabförmigen Elementen vorliegt, die in wenigstens eine Ausnehmung (4) in dem Einbauraum eingesetzt sind und mittels wenigstens eines Fixierelements fixiert sind, das die Kugeln bzw. die stabförmigen Elemente lediglich auf einem Teil ihrer Fläche berührt.

2. Endoskop nach Anspruch 1,
**dadurch gekennzeichnet, daß** das wenigstens eine Fixierelement ein O-Ring (6) ist, der das Trockenmittel in der Ausnehmung fixiert.

3. Endoskop nach Anspruch 1,
**dadurch gekennzeichnet, daß** das wenigstens eine Fixierelement ein Behältnis ist, dessen Wandung feuchtepermeabel ist.

4. Endoskop nach Anspruch 3,
**dadurch gekennzeichnet, daß** die Wandung des Behältnisses aus einer Membran besteht.

5. Endoskop nach einem der Ansprüche 1 bis 4,
**dadurch gekennzeichnet, daß** das Trockenmittel im Bereich des Okulartrichters (1) angeordnet ist.

6. Endoskop nach einem der Ansprüche 1 bis 5,
**dadurch gekennzeichnet, daß** das Trockenmittel ein Silica-Gel oder eine poröse Keramik ist.

7. Endoskop nach einem der Ansprüche 1 bis 6,
**dadurch gekennzeichnet, daß** in dem Einbauraum der Optik eine Einrichtung zur Messung der Feuchte vorgesehen ist.

8. Endoskop nach Anspruch 7,
**dadurch gekennzeichnet, daß** die Einrichtung zur Messung der Feuchte einen Farbindikator aufweist, dessen Farbei bei einem bestimmten Feuchtigkeitsgrad reversibel oder irreversibel umschlägt, und der in einem Fenster von außen sichtbar ist.

9. Endoskop nach einem der Ansprüche 1 bis 8,
**dadurch gekennzeichnet, daß** das Fixierelement derart ausgebildet ist, daß das Trockenmittel im Rahmen eines Endoskop-Service austauschbar ist.

## Claims

1. Endoscope comprising an optical endoscope system and a drying agent introduced into and fixed in the mounting space for the optical endoscope system, **characterised in that** said drying agent is present in the form of a plurality of spheres (5) and/or rod-shaped elements inserted into at least one recess (4) in said mounting space and fixed by means of at least one fixing element that contacts said spheres or said rod-shaped elements merely on one part of their surface.

2. Endoscope according to Claim 1,
**characterised in that** said at least one fixing element is an O-ring (6) that fixes said drying agent in said recess.

3. Endoscope according to Claim 1,
**characterised in that** said at least one fixing element is a recipient whose wall is permeable to moisture.

4. Endoscope according to Claim 3,
**characterised in that** the wall of said recipient consists of a membrane.

5. Endoscope according to any of the Claims 1 to 4,
**characterised in that** said drying agent is disposed in the zone of the eyepiece funnel (1).

6. Endoscope according to any of the Claims 1 to 5,
**characterised in that** said drying agent is a silica gel or a porous ceramic material.

7. Endoscope according to any of the Claims 1 to 6,
**characterised in that** a moisture measuring means is provided in the mounting space for the optical system.

8. Endoscope according to Claim 7,
**characterised in that** said moisture measuring means includes a colour indicator whose colour changes at a defined moisture level in a reversible or irreversible manner and which is visible in a window from the outside.

9. Endoscope according to any of the Claims 1 to 8,
**characterised in that** said fixing element is so configured that the drying agent is replaceable on the occasion of endoscope servicing.

## Revendications

1. Endoscope comprenant un système optique d'endoscope et un agent de séchage, introduit et fixé dans l'espace d'emplacement pour le système optique d'endoscope,
**caractérisé en ce que** ledit agent de séchage est présent sous forme d'une pluralité de sphères (5) et/ou des éléments sous forme de barres insérés dans au moins une gorge (4) dans ledit espace d'emplacement, en étant fixé moyennant au moins un élément fixeur, qui ne se trouve en contact avec lesdites sphères ou lesdits éléments sous forme de barres que sur une partie de leur surface.

2. Endoscope selon la revendication 1,
**caractérisé en ce que** ledit au moins un élément fixeur est un rond en O (6) qui sert à fixer ledit agent de séchage dans ladite gorge.

3. Endoscope selon la revendication 1,
**caractérisé en ce que** ledit au moins un élément fixeur est un récipient, dont la paroi est perméable à l'humidité.

4. Endoscope selon la revendication 3,
**caractérisé en ce que** la paroi dudit récipient est constitué par une membrane.

5. Endoscope selon une quelconque des revendications 1 à 4,
**caractérisé en ce que** ledit agent de séchage est disposé dans la zone du cône d'oculaire (1).

6. Endoscope selon une quelconque des revendications 1 à 5,
**caractérisé en ce que** ledit agent de séchage est un gel de silice ou un matériau céramique poreux.

7. Endoscope selon une quelconque des revendications 1 à 6,
**caractérisé en ce qu'**un moyen hydrométrique est disposé dans l'espace d'emplacement pour le système optique.

8. Endoscope selon la revendication 7,
**caractérisé en ce que** ledit moyen hydrométrique comprend un indicateur coloré dont la couleur subit un virage à un niveau d'humidité défini d'une manière réversible ou non réversible et qui set visible, dans une fenêtre, de l'extérieur.

9. Endoscope selon une quelconque des revendications 1 à 8,
**caractérisé en ce que** ledit élément fixeur est configuré de façon, que l'agent de séchage soit échangé au temps de service de l'endoscope.
